(19) Europäisches Patentamt
European Patent Office
Office européen des brevets

(11) **EP 3 527 135 A1**

(12) **EUROPEAN PATENT APPLICATION**
published in accordance with Art. 153(4) EPC

(43) Date of publication:
**21.08.2019 Bulletin 2019/34**

(51) Int Cl.:
**A61B 5/1455** (2006.01)

(21) Application number: **16918590.7**

(22) Date of filing: **14.10.2016**

(86) International application number:
**PCT/JP2016/080534**

(87) International publication number:
**WO 2018/070035 (19.04.2018 Gazette 2018/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujita Medical Instruments Co., Ltd. Tokyo 113-0033 (JP)**

(72) Inventors:
• **Maeda, Hironobu**
**Tokyo 113-0033 (JP)**
• **Yamamura, Haruo**
**Tokyo 113-0033 (JP)**

(74) Representative: **Haseltine Lake Kempner LLP Redcliff Quay**
**120 Redcliff Street**
**Bristol BS1 6HU (GB)**

(54) **BIOLOGICAL INFORMATION MEASUREMENT INSTRUMENT**

(57) Provided is a biological information measurement instrument including a flexible substrate having an elongated shape, a light emitting unit which radiates near-infrared light, a light receiving unit which detects light, and a light shielding film, in which the light emitting unit is on a surface side of the flexible substrate and provided in a first recess formed on the flexible substrate, the light receiving unit is on the surface side of the flexible substrate, spaced apart from the first recess, and provided in a second recess formed on the flexible substrate, the light shielding film has a light-transmitting property at portions at which the light receiving unit and the light-emitting unit are positioned, and is provided on the surface side of the flexible substrate, and the light shielding film, a light emitting surface of the light emitting unit, and a light receiving surface of the light receiving unit are configured to form one surface.

**FIG 1**

EP 3 527 135 A1

**Description**

[Technical Field]

The present invention relates to a biological information measurement instrument capable of acquiring biological information of a human body.

[Background Art]

Conventionally, there is a diagnostic technique in which disposable sensors are used as a biological information measurement instrument when diagnosing a site of a lesion such as cancer without incising a human body. Disposable sensors are used to estimate the oxygen saturation or an amount of change in hemoglobin concentration of the blood in a human body, for example, in the brain. Such disposable sensors include a light emitting element that emits near-infrared light and a light receiving element that receives near-infrared light from the light emitting element that has passed through a human body, and outputs the sensing data from the light receiving element.

The oxygen saturation or an amount of change in hemoglobin of blood can be estimated by determining an amount of attenuation of light and determining an amount of light absorbed by hemoglobin from the sensing data. This technique utilizes Lambert-Beer's law in which an amount of absorbed light is proportional to the product of incident light and a concentration of solute. Hemoglobin includes oxyhemoglobin and deoxyhemoglobin, each having different light absorption spectra. Thus, the light emitting element emits light of different wavelengths, and the light receiving element determines an amount of light absorbed through a measurement object and obtains an absorbance from the sensing data, thereby the oxygen saturation being able to be estimated (calculated) by using a ratio therebetween. Also, change in an amount related to light absorption of total hemoglobin can be estimated (calculated) by using light of a wavelength at which light absorption coefficients of both oxyhemoglobin and deoxyhemoglobin are equal and measuring the amount of light absorption thereof. An example of such a disposable sensor is disclosed in Patent Literature 1.

[Citation List]

[Patent Literature]

[Patent Literature 1]
United States Patent Application Publication No. 2015/0126831A1
[Patent Literature 2]
United States Patent Application Publication No. 2013/0317330A1

[Summary of Invention]

[Technical Problem]

Incidentally, the above-described disposable sensor is based on absorbance of light by hemoglobin and has a problem in that oxygen saturation or an amount of change thereof may not be able to be accurately calculated when other light, that is, external light enters. In the above-described Patent Literature 1, in order to cope with this, a light emitting unit and a light receiving unit are provided on a transparent substrate to carry out light emitting and light receiving through the transparent substrate, and a carbon coating is applied to portions other than those at which the light emitting unit and the light receiving unit are positioned to deal with the problem. However, use of a transparent substrate and application of a carbon coating onto a transparent substrate cause a problem that the cost per unit then increases.

The present invention has been made in view of the above-described problems, and it is an objective of the present invention to provide a biological information measurement instrument configured such that external light is inhibited from entering a light receiving unit as much as possible at the time of use without using a transparent substrate coated with a carbon coating.

[Solution to Problem]

In order to solve the problems described above, a biological information measurement instrument according to the present invention includes a flexible substrate having an elongated shape, a light emitting unit which radiates near-infrared light, a light receiving unit which detects light, and a light shielding film, in which the light emitting unit is on a surface side of the flexible substrate and provided in a first recess formed on the flexible substrate, the light receiving unit is on the surface side of the flexible substrate, spaced apart from the first recess, and provided in a second recess formed on the flexible substrate, the light shielding film has a light-transmitting property at portions at which the light receiving unit and the light-emitting unit are positioned, and is provided on the surface side of the flexible substrate, and the light shielding film, a light emitting surface of the light emitting unit, and a light receiving surface of the light receiving unit are configured to form one surface.

The biological information measurement instrument is used by being attached to a human body so that the light emitting surface and the light receiving surface are brought into contact with the human body, and the light receiving unit may receive near-infrared light emitted from the light emitting unit through the human body.

In the biological information measurement instrument, the light shielding film may include openings at portions with which the light emitting surface of the light emitting unit and the light receiving surface of the light receiving unit are in contact.

In the above biological information measurement instrument, the light shielding film may have a shape extending at least a predetermined distance or more from the openings to such an extent that light other than the light emitted from the light emitting unit is able to be shielded during the measurement instrument being attached to the human body.

In the biological information measurement instrument, an impact absorbing material may be provided on a back surface of the flexible substrate.

The biological information measurement instrument may further include an output unit that outputs information on the light received by the light receiving unit.

In the biological information measurement instrument, the first recess and the second recess may be formed by bending the flexible substrate.

[Advantageous Effects of Invention]

The biological information measurement instrument according to one aspect of the present invention includes the recesses formed on the flexible substrate, the light emitting unit and the light receiving unit provided therein, and the light shielding film provided on the flexible substrate, and thereby it is possible to provide a biological information measurement instrument in which external light is inhibited from entering the light receiving unit as far as possible at the time of use without using a transparent substrate coated with a carbon coating.

[Brief Description of Drawings]

Fig. 1(a) is a plan view of a biological information measurement instrument 100.

Fig. 1(b) is a cross-sectional view of a state of the biological information measurement instrument 100 in which a light emitting unit and a light receiving unit are not provided.

Fig. 1(c) is a cross-sectional view of the biological information measurement instrument 100.

Fig. 2 is a perspective view of the biological information measurement instrument 100.

Fig. 3 is an exploded perspective view of the biological information measurement instrument 100.

Fig. 4 is a flowchart illustrating an operation of the biological information measurement instrument 100.

Figs 5(a) to 5(c) are views illustrating configuration examples of the biological information measurement instrument 100.

Fig. 6 is a view illustrating an example of use of the biological information measurement instrument 100.

Figs. 7(a) and 7(b) are cross-sectional views illustrating configurations of conventional biological information measurement instruments 700 and 710.

[Description of Embodiments]

<Findings obtained by the inventors>

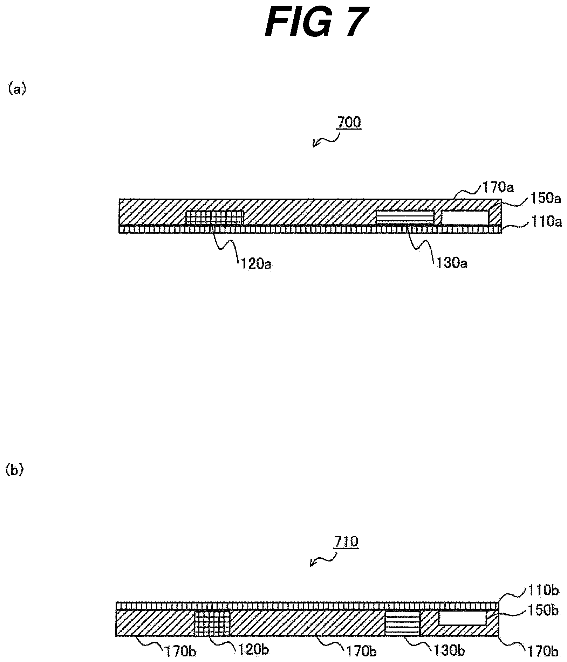

One of such disposable sensors is disclosed in Patent Literature 1. Fig. 7(a) illustrates a side view of a disposable sensor 700 disclosed in Patent Literature 1. In the disposable sensor 700 illustrated in Fig. 7(a), a light emitting unit 120a, a light receiving unit 130a, and an output unit 150b are provided on one surface of a transparent flexible substrate 110a. The light emitting unit 120a is provided such that a light emitting surface thereof faces the transparent flexible substrate 110a. Also, the light receiving unit 130a is also provided such that a light receiving surface thereof faces the transparent flexible substrate 110a. Further, an impact absorbing material 170a for protecting the light emitting unit 120a and the light receiving unit 130a is provided to cover the light emitting unit 120a and the light receiving unit 130a as illustrated in Fig. 7(a). The disposable sensor 700 illustrated in Fig. 7(a) is used with a lower side in the drawing being brought into contact with a human body. According to the disposable sensor according to Patent Literature 1, light emitted from the light emitting unit 120a passes through the transparent flexible substrate 110a and is radiated to a human body, the light that has passed through the human body passes through the transparent flexible substrate 110a and is received by the light receiving unit 130a, and thereby sensing can be performed. Thus, the flexible substrate 110a is configured as a multilayer substrate in which a carbon coating is applied other than at positions at which the light emitting unit 120a and the light receiving unit 130a are disposed in order to shield light from the outside.

However, in a case of such a configuration, that is, in a case of applying a carbon coating onto a transparent flexible substrate, there is a problem that the cost per unit is higher than in a case of simply using an opaque flexible substrate.

Therefore, as a disposable sensor of another aspect not using such a transparent flexible substrate, there is one illustrated in Fig. 7(b). As illustrated in Fig. 7(b), a disposable sensor 710 according to another aspect includes a light emitting unit 120b, a light receiving unit 130b, an output unit 150b, and an impact absorbing material 170b which are provided on one surface of a flexible substrate 110b. The impact absorbing material 170b includes openings for exposing the light emitting unit 120b and the light receiving unit 130b and is realized using a flexible foamed rubber or the like.

Incidentally, when such a disposable sensor 710 is attached to a human body, it is desirable that a light emitting surface of the light emitting unit 120 and a light receiving surface of the light receiving unit 130 be brought into uniform contact with the human body as described above, but a surface of a human body is generally formed of a curved surface. Therefore, although the disposable sensor 710 is used by being bent at the time of use, there is a possibility that wrinkles may occur at a position of the light receiving unit 130b at that time. Then, the inventors have found that there is a possibility that calculation of absorbance used for calculation of oxygen saturation or concentration of hemoglobin may be erroneous due to external light entering from the wrinkles and becoming noise in detection of the light receiving unit.

Therefore, the inventors have invented a biological information measurement instrument in which such wrinkles do not occur as far as possible when the biological information measurement instrument is attached to the human body. Hereinafter, a biological information measurement instrument will be described with reference to the drawings.

<Embodiment>

<Configuration>

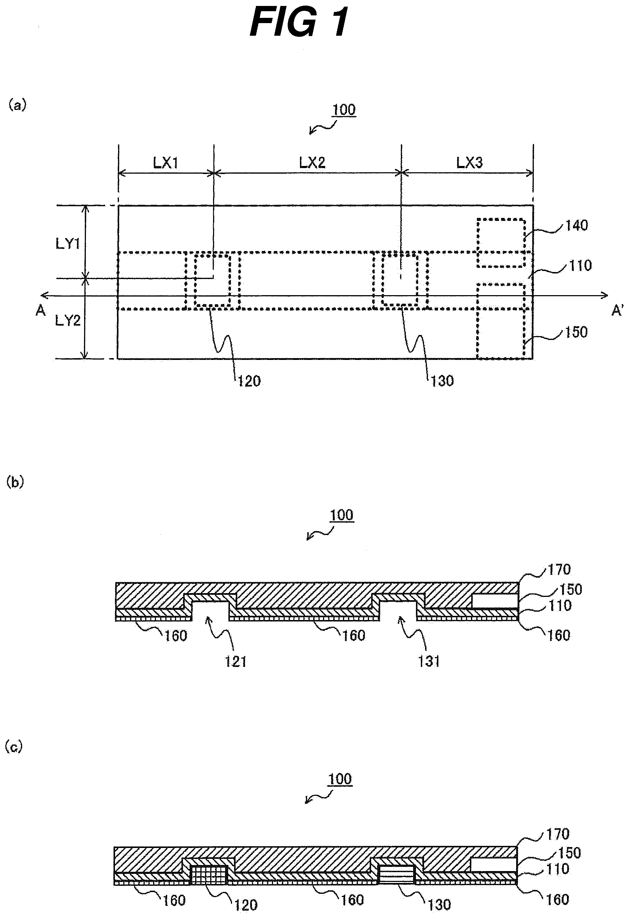

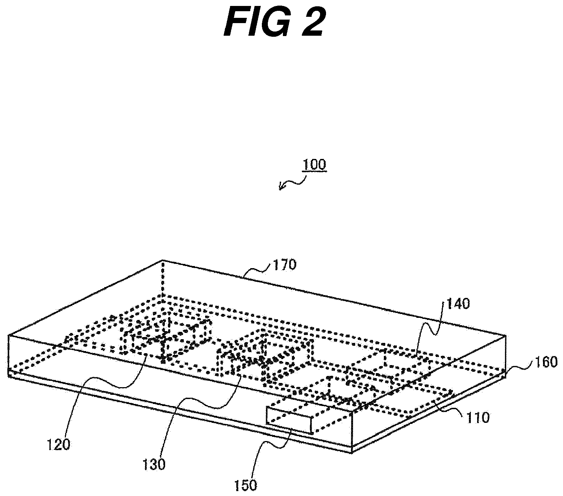

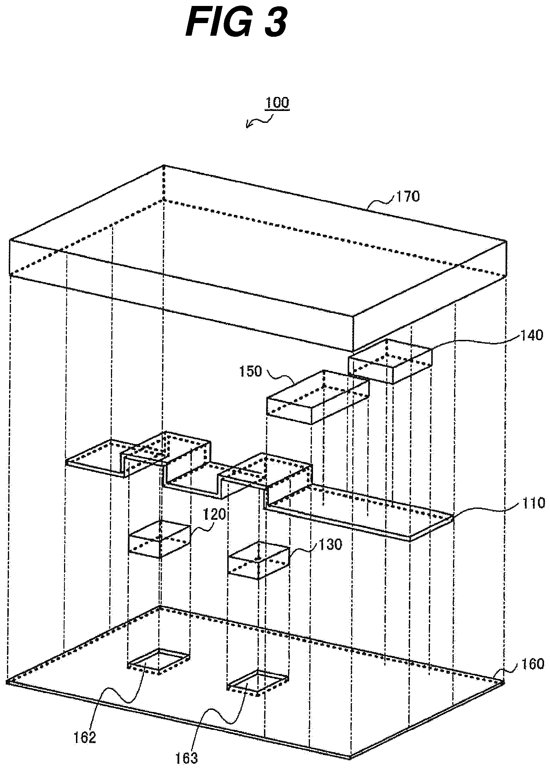

A configuration of a biological information measurement instrument 100 according to the present invention will be described using Figs. 1 to 3. Fig. 1(a) is a plan view of the biological information measurement instrument 100 from an impact absorbing material 170 side. Fig. 1(b) is a side view of the biological information measurement instrument 100 illustrating a state in which a light emitting unit 120 and a light receiving unit 130 are not provided. Fig. 1(c) is a side view of the biological information measurement instrument 100 illustrating a state in which the light emitting unit 120 and the light receiving unit 130 are provided. Also, Fig. 2 is a perspective view of the biological information measurement instrument 100, and Fig. 3 is an exploded perspective view of the biological information measurement instrument 100.

As illustrated in Figs. 1 to 3, the biological information measurement instrument 100 according to the present invention includes a flexible substrate 110, the light emitting unit 120, and the light receiving unit 130.

The flexible substrate 110 is formed in an elongated shape. As illustrated in Fig. 2, the flexible substrate 110 includes a first recess 121 formed by bending the flexible substrate 110 and a second recess 131 formed to be spaced apart from the first recess 121. The flexible substrate 110 uses a double-sided substrate.

As illustrated in Fig. 3, the light emitting unit 120 is provided in the first recess 121. The light emitting unit 120 is a light emitting element that emits near-infrared light. Although the light emitting unit 120 may be realized by, for example, a light emitting diode (LED), an element other than an LED may also be used. The light emitting unit 120 is disposed such that a light emitting surface thereof is exposed to the outside, that is, toward a lower side of the drawing in Fig. 1(c). The light emitting unit 120 is a light emitting element capable of controlling a light emission wavelength and emits light at a designated light emission wavelength in accordance with an instruction from a control unit 140. Here, the light emitting unit 120 emits near-infrared light having any one of wavelengths of 770 nm, 805 nm, and 870 nm. The light emitting unit 120 may be realized by an LED that emits light by switching between these wavelengths or may be realized by providing a plurality of LEDs emitting light at each wavelength. Also, these wavelengths are examples, and other wavelengths may also be used.

Also, the light receiving unit 130 is provided in the second recess 131. The light receiving unit 130 is a light sensor that detects light. Although the light receiving unit 130 may be realized by, for example, a photodiode, an element other than a photodiode may also be used. A photodiode is a photovoltaic-type element, but there are other photodetectors of types called a photoconductive-type or a thermal effect-type. The light receiving unit 130 is disposed such that a light receiving surface thereof is exposed to the outside, that is, toward the lower side of the drawing in Fig. 1(c).

As illustrated in Fig. 1(c), the biological information measurement instrument 100 is configured such that a surface of a light shielding film 160, the light emitting surface of the light emitting unit 120, and the light receiving surface of the light receiving unit 130 are formed on one surface. The one flat surface may be a planar surface as illustrated in Fig. 1(c) in some cases or the one flat surface may also be a curved surface in some cases when it is used by being bent at the time of use. Thus, since the whole of one surface can be brought into contact with a human body, it is possible to have a configuration in which external light cannot easily enter at the time of use.

Specifically, the biological information measurement instrument 100 is used by being brought into contact with a human body and detect biological information of the human body at the contact region, for example, oxygen saturation or an amount of change of hemoglobin. The biological information measurement instrument 100 may be, for example, a disposable sensor. Therefore, when the biological information measurement instrument 100 is attached to a human body, it is used by being attached to the human body along the curved surface of the skin. In order to obtain such biological information, the biological information measurement instrument 100 is attached to a portion (for example, the forehead, an affected site, or the like of the human body) from which biological information of the human body is desired to be obtained, and near-infrared light is emitted toward the human body from the light emitting unit 120. The light receiving unit 130 detects the near-infrared light emitted from the light emitting unit 120 through the human body. It is a well-known fact that oxygen saturation in blood or an amount of change related to light absorption of hemoglobin can be detected by analyzing near-infrared light. Specifically, utilizing Lambert-Beer's law in which an amount of absorbed light is proportional to the product of incident light and a concentration of solute, the measurement is performed by using differences in light absorption spectra between oxyhemoglobin and deoxyhemoglobin. That is, an absorbance is obtained from the light having passed through a contact region at different wavelengths and being detected by the light receiving unit 130. From the absorbance, the oxygen saturation can be calculated.

The control unit 140 is a processor having a function of controlling each unit of the biological information measurement instrument 100. The control unit 140 operates according to a preset program and, specifically, has the following functions. The control unit 140 calculates an oxygen saturation in blood on the basis of the sensing data transmitted from the light receiving unit 130. Also, the control unit 140 calculates an amount of change related to the light absorption of hemoglobin on the basis of the sensing data transmitted from the light receiving unit 130. The control unit 140 outputs the calculated information to an external device via an output unit 150. The information may be referred to as a hemoglobin index in some cases.

Here, a method of calculating the oxygen saturation will be briefly described. When an absorbance of oxyhemoglobin is KHbO2, an absorbance of deoxyhemoglobin is KHb, an oxygen saturation is rSO2 = HbO2/(HbO2+Hb), an optical path length is d, and a concentration of solute is C, an absorbance K at a certain wavelength can be calculated from the following Expression (1).

$$K = (rSO2 \times KHbO2 + (1-rSO2) \times KHb)Cd \cdots (1)$$

Then, absorbances KR and KIR are calculated from detection signals by the light receiving unit 130 at two wavelengths of a wavelength R (for example, 770 nm) and a wavelength IR (for example, 870 nm). Then, the control unit 140 calculates the oxygen saturation on the basis of the ratio KR/KIR = R/IR and a relational expression representing a relationship between the ratio and the oxygen saturation previously stored by the control unit 140.

Also, the control unit 140 calculates an amount of change in hemoglobin by calculating the absorbance at a wavelength (805 nm) at which light absorption coefficients of both oxyhemoglobin and deoxyhemoglobin are equal. This is because it is known that an amount of light absorption correlates with an amount of change in hemoglobin, and it is based on the fact that an amount of change in absorbance can be taken as an amount of change in hemoglobin.

The output unit 150 is a connector for connecting to an external device, and is an interface having a function of outputting information transmitted from the control unit 140 to the connected external device. The output unit 150 transmits the information by being connected to the external device using wires. The output unit 150 performs communication in accordance with a predetermined communication protocol, and performs communication in accordance with, for example, the USB (IEEE 1394) standard. Further, the communication protocol is not limited to the USB standard as a matter of course. If a connector which is available on the market is used for the output unit 150, manufacture thereof can be facilitated and usability of the biological information measurement instrument 100 can be improved.

As illustrated in Figs. 1(b) and 1(c), Fig. 3, or the like, the light shielding film 160 is provided on a surface of the flexible substrate 110. The light shielding film 160 is adhered to the surface of the flexible substrate 110 using, for example, an adhesive such as double-sided tape. As illustrated in Fig. 3, the light shielding film 160 includes an opening 162 and an opening 163. The opening 162 is provided at a position facing the light emitting unit 120, and the opening 163 is provided at a position facing the light receiving unit 130. Thereby, the light emitting surface of the light emitting unit 120 and the light receiving surface of the light receiving unit 130 are exposed to the outside and have a structure that can be brought into contact with a contact region of a human body. The light shielding film 160 has a length extending at least a predetermined distance or more from the opening 162 and the opening 163. Here, the predetermined distance is a length such that external light does not enter from a gap between the human body and the biological information measurement instrument 100 when the biological information measurement instrument 100 is attached to the human body. That is, when the biological information measurement instrument 100 is attached in close contact with the human body, the light shielding film 160 is formed to have such a size as to be able to shield light other than the light emitted from the light emitting unit 120. As the light shielding film 160, a black film having flexibility and made as thin as possible is used as an example, but the present invention is not limited thereto as long as a film can sufficiently shield light. Here, as the light shielding film 160, one having a thickness of about 0.2 mm is used. This thickness of the light shielding film 160 is an example, and the thickness may be other than this.

As illustrated in Fig. 1(a), the light shielding film 160 is configured to have distances of LX1, LY1, and LY2 from a center of the light emitting unit 120 to an end portion thereof. Accordingly, distances from a center of the light receiving unit 130 to the end portion of the light shielding film 160 also are distances of LY1 and LY2. As described above, the distance has a length such that external light does not reach the light receiving unit 130. Also, a distance from the center of the light emitting unit 120 to the center of the light receiving unit 130 is LX2, and a distance from the light receiving unit

130 to an end portion of the light shielding film 160 is LX3. The distances LX2 and LX3 are determined according to practical use. As a description of one specific example, for example, when the biological information measurement instrument 100 is used by being attached to a forehead of a human body, the lengths are set to about LX1 = 15 mm, LX2 = 30 mm, LX3 = 45 mm, LY1 = 12.5 mm, and LY2 = 12.5 mm. Lengths slightly greater or less than the lengths as above may also be used. With such lengths, it is possible to have a configuration in which a feeling of discomfort is not imparted to a user as far as possible at the time of attachment to the human body, external light does not enter, and furthermore, a cable connected to the output unit 150 does not disturb the measurement. Further, when used by being attached to the forehead of the human body, the biological information measurement instrument 100 is preferably used as a pair thereof with a bilaterally symmetrical configuration.

Also, in the biological information measurement instrument 100, the impact absorbing material 170 is provided on a back surface of the flexible substrate 110. The impact absorbing material 170 is a member that absorbs an impact to the control unit 140, the output unit 150, or the like from the outside, and can be realized by, for example, a sponge, a piece of rubber, or the like, but the present invention is not limited thereto. The impact absorbing material 170 is adhered to the back surface of the flexible substrate 110 using, for example, an adhesive such as double-sided tape. The impact absorbing material 170 may be processed to have a shape conforming to irregularities of shapes of the flexible substrate 110, the output unit 150, or the like, or may be adhered by being pressed from above using its flexibility in a state of not being processed.

<Example of method of manufacturing biological information measurement instrument 100>

An example of a method of manufacturing the biological information measurement instrument 100 will be briefly described.

First, each part is formed on the flexible substrate 110. As the flexible substrate 110, a double-sided substrate having a thickness of 0.1 to 0.2 mm as described above is prepared. Further, this thickness is merely a reference, and a thickness outside this range may also be used. An LED as the light emitting unit 120, a photodiode as the light receiving unit 130, a processor as the control unit 140, and a connector as the output unit 150 are mounted on the flexible substrate 110 at positions illustrated in Fig. 1(a).

Next, the flexible substrate 110 is bent and formed so that the flexible substrate 110 on which each part is mounted has a shape as illustrated in Fig. 3. That is, the flexible substrate 110 is bent and formed so that the first recess 121 and the second recess 131 illustrated in Fig. 1(b) are formed while wirings inside the flexible substrate 110 are not disconnected. Then, the flexible substrate 110 is adhered to the light shielding film 160 as a sensor base. In the light shielding film 160, the openings 162 and 163 are provided at portions at which the light emitting unit 120 and the light receiving unit 130 are positioned and a double-sided tape for adhesion is attached thereto in advance. Also, a double-sided tape for being attached to a human body is adhered to a side of the light shielding film 160 opposite to the side to which the flexible substrate 110 after the parts are mounted is adhered, that is, to a side being brought into contact with the human body.

After adhering the flexible substrate 110 with the parts mounted to the light shielding film 160, a cover made of foamed rubber serving as the impact absorbing material 170 is adhered thereto, and then the biological information measurement instrument 100 is manufactured.

In this way, the biological information measurement instrument 100 in which mounting by a machine is facilitated can be provided. Moreover, a connector which is available on the market can be used for the connector and can be disposed at a position at which a cable thereof does not become a disturbance in practical use when the biological information measurement instrument 100 is attached to the human body.

Further, respective procedures in the processing described above may be performed in a reverse order as long as the procedures allow the final shape illustrated in Fig. 2 to be formed. For example, although formation of the flexible substrate 110 is performed after the parts are mounted on the flexible substrate 110 in the above description, the same result can be obtained even when the procedures are reversed. As described above, when the same result can be obtained, the order of processing respective procedures may be reversed.

<Operation>

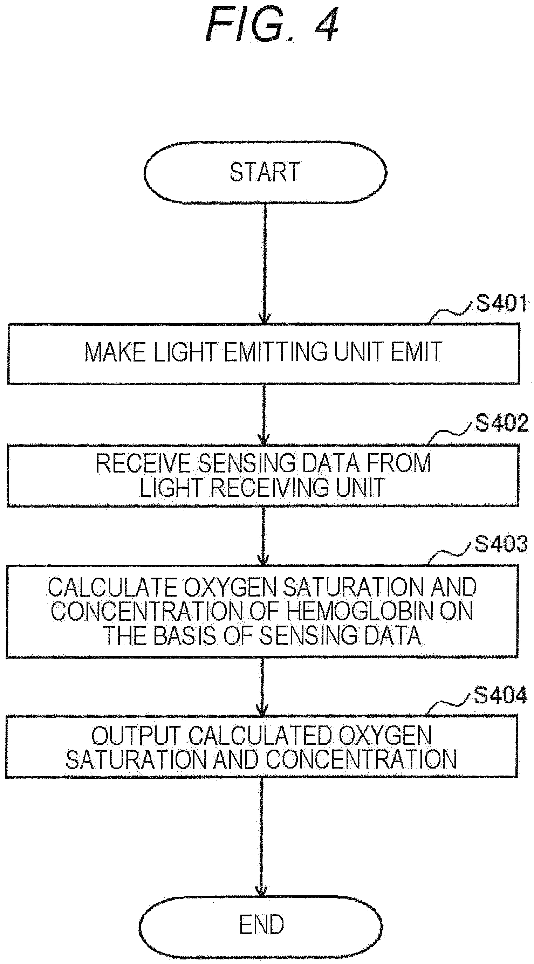

Fig. 4 is a flowchart illustrating an operation of the biological information measurement instrument 100.

As illustrated in Fig. 4, the control unit 140 of the biological information measurement instrument 100 causes the light emitting unit 120 to emit light at a predetermined wavelength (step S401). Here, predetermined wavelengths include 770 nm, 805 nm, and 870 nm, and the light emitting unit 120 causes each of corresponding LEDs to emit light for a predetermined time. Regarding the wavelengths, a wavelength other than the wavelengths described above may also be used as long as the oxygen saturation described above can be calculated thereby.

The control unit 140 receives sensing data from the light receiving unit 130 that has received near-infrared light of each wavelength. Here, the sensing data indicates an amount of light received by the light receiving unit 130 (step S402).

The control unit 140 calculates the oxygen saturation in blood on the basis of each amount of light transmitted from the light receiving unit 130 according to the wavelength of light emitted by the light emitting unit 120. Also, an amount of change in hemoglobin is calculated on the basis of the calculated oxygen saturation (step S403).

The control unit 140 outputs information on the calculated oxygen saturation and the amount of change in hemoglobin to an external device via the output unit 150 (step S404).

In this way, the output information of various types is displayed on a monitor provided in the external device or a monitor connected to the external device. At this time, the external device may process the information of various types output from the biological information measurement instrument 100 and then perform display.

<Summary>

As described above, in the biological information measurement instrument 100, by providing the first recess 121 and the second recess 131 in the flexible substrate 110, measurement of biological information can be realized without using a carbon-coated transparent substrate. Also, by forming the light shielding film 160, the light emitting surface of the light emitting unit 120, and the light receiving surface of the light receiving unit 130 on one surface, it is possible to provide a biological information measurement instrument in which wrinkles cannot easily occur when it is attached to a human body and external light does not easily enter. Accordingly, a biological information measurement instrument capable of stable and accurate measurement can be provided.

<Supplement>

The biological information measurement instrument according to the embodiment described above is not limited to the embodiment described above and may be realized by another method as a matter of course. Hereinafter, modified examples of various types will be described.

(1) In the embodiment described above, an oxygen saturation in blood and an amount of change in hemoglobin are calculated in the control unit 140 of the biological information measurement instrument 100 and then output from the output unit 150, but the present invention is not limited thereto.

The biological information measurement instrument 100 may have a configuration in which sensing data from the light receiving unit 130 is output to an external device without change and the oxygen saturation in blood and the amount of change in hemoglobin are calculated in the external device.

(2) Although not particularly described in the embodiment described above, a cable connected to an external device may be connected to the output unit 150 and the biological information measurement instrument 100 is driven by receiving power supplied from the external device.

(3) In the embodiment described above, the opening 161 and the opening 162 are provided in the light shielding film 160, but instead of this configuration, the light shielding film 160 may be configured to have a transparent film only at positions at which the opening 161 and the opening 162 are positioned. This can be realized by, for example, spraying a black ink or the like on the transparent film other than at portions at which the openings 161 and the openings 162 are positioned.

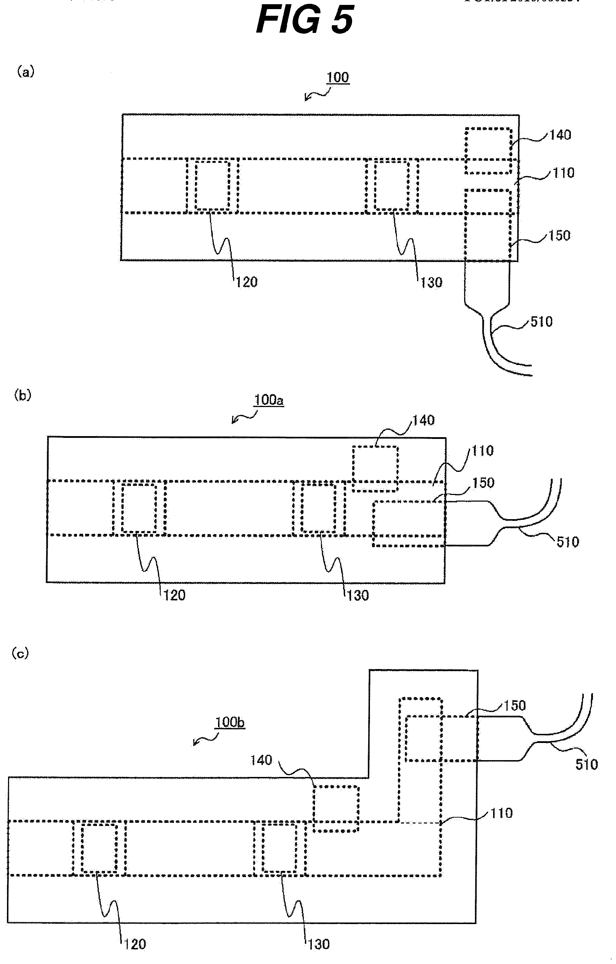

(4) In the embodiment described above, the output unit 150 is disposed at a right angle with respect to the flexible substrate 110, but this is not limited thereto. Fig. 5(a) illustrates a state in which a cable is connected to the biological information measurement instrument 100 described in the embodiment described above. In contrast, the output unit 150 may be disposed along the flexible substrate 110. That is, a configuration in which the output unit 150 is disposed as illustrated in Fig. 5(b) so that a cable can be connected thereto may be used.

Alternatively, the biological information measurement instrument 100 may be configured to further extend to have an L-shaped shape as illustrated in Fig. 5(c). The biological information measurement instrument 100 may have any shape as long as it is configured such that the light emitting unit 120 and the light receiving unit 130 are brought into contact with an object to be measured and light from the outside does not enter the light receiving unit 130 during measurement, and may be configured to have a shape such as, for example, a circle, a U-shape, or a crank. By changing the shape of the biological information measurement instrument 100, it is possible to provide the biological information measurement instrument 100 having a shape suitable for an object to be measured or a shape facilitating measurement.

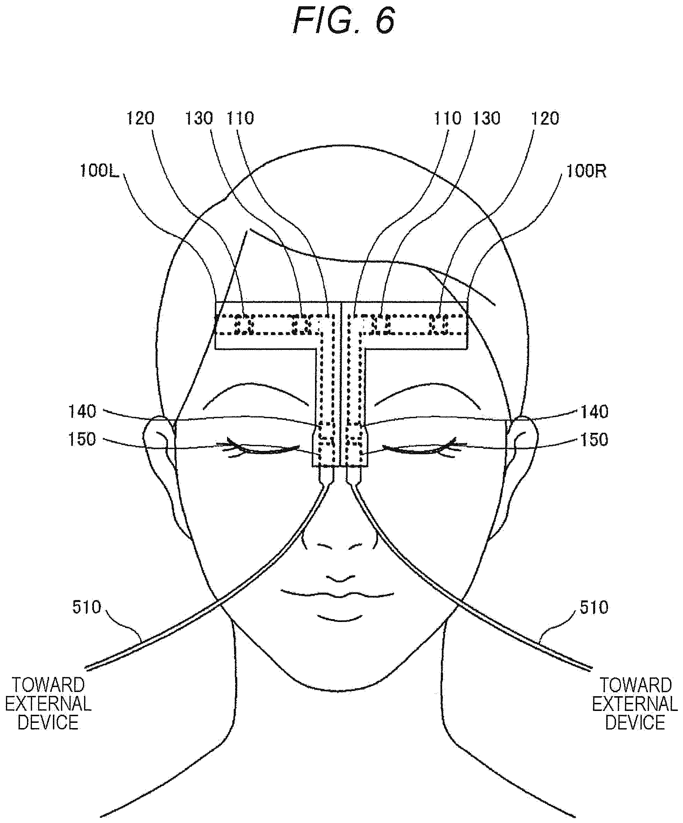

(5) Although not described in detail in the embodiment described above, an example of use of the biological information measurement instrument 100 will be described here. Fig. 6 is a view illustrating an example of use of the biological information measurement instrument 100. As illustrated in Fig. 6, the biological information measurement instrument 100 uses two biological information measurement instruments 100L and 100R having a bilaterally symmetrical configuration that are attached to a forehead of a human body. Further, the biological information measurement instrument 100L and the biological information measurement instrument 100R may be integrally molded to be configured as a T-shaped measurement instrument.

Fig. 6 illustrates an example in which a pair of left and right biological information measurement instruments 100 configured in an L-shape is used by being attached to a head of the human body. As illustrated in Fig. 6, the biological information measurement instruments 100 is attached so that the light emitting unit 120 and the light receiving unit 130 are brought into contact with the forehead of the human body. The flexible substrate 110 has a shape extending along a bridge of a nose of the human body, and the output unit 150 is configured at an end portion thereof. Further, the control unit 140 is also configured on the flexible substrate 110. A cable 510 is connected to the output unit 150 of the flexible substrate 110. Although not illustrated, the cable 510 is connected to an external device to provide a communication path from the biological information measurement instrument 100 to the external device. In this way, the biological information measurement instrument 100 can be attached to a head of the human body, calculate an oxygen saturation and a concentration of hemoglobin in the brain, and output the calculated information to the external device for display.

(6) In the embodiment described above, as a method of calculating and outputting the oxygen saturation and the concentration of hemoglobin in the biological information measurement instrument, a processor (control unit 140) of the biological information measurement instrument executes a biological information calculation program or the like and then performs output, but this may also be realized by a logic circuit (hardware) or a dedicated circuit formed in an integrated circuit (an integrated circuit (IC) chip, a large scale integration (LSI)), or the like in the biological information measuring instrument. Also, these circuits may be realized by one or more integrated circuits, and the functions of the plurality of functional units described in the embodiment described above may be realized by one integrated circuit. The LSI may be called a VLSI, a super LSI, an ultra LSI, or the like depending on a degree of integration.

Also, the biological information measurement program described above may be recorded in a recording medium readable by a processor, and a "non-temporary tangible medium" such as a tape, a disk, a card, a semiconductor memory, or a programmable logic circuit can be used for the recording medium. Further, the biological information measurement program described above may be supplied to the processor described above through an arbitrary transmission medium (communication network, broadcast wave, or the like) capable of transmitting the biological information measurement program. The present invention can also be realized in a form of a data signal embedded in a carrier wave in which the biological information measurement program is embodied by electronic transmission.

[Reference Signs List]

100 Biological information measurement instrument
110 Flexible substrate
120 Light emitting unit
121 First recess
130 Light receiving unit
131 Second recess
140 Control unit
150 Output unit
170 Impact absorbing material

**Claims**

1. A biological information measurement instrument comprising:

   a flexible substrate having an elongated shape;
   a light emitting unit which radiates near-infrared light;
   a light receiving unit which detects light; and
   a light shielding film, wherein
   the light emitting unit is on a surface side of the flexible substrate and provided in a first recess formed on the flexible substrate,
   the light receiving unit is on the surface side of the flexible substrate, spaced apart from the first recess, and provided in a second recess formed on the flexible substrate,
   the light shielding film has a light-transmitting property at portions at which the light receiving unit and the light emitting unit are positioned, and is provided on the surface side of the flexible substrate, and
   the light shielding film, a light emitting surface of the light emitting unit, and a light receiving surface of the light receiving unit are configured to form one surface.

2. The biological information measurement instrument according to claim 1 used by being attached to a human body so that the light emitting surface and the light receiving surface are brought into contact with the human body, wherein the light receiving unit receives near-infrared light emitted from the light emitting unit through the human body.

3. The biological information measurement instrument according to claim 2, wherein the light shielding film includes openings at portions with which the light emitting surface of the light emitting unit and the light receiving surface of the light receiving unit are in contact.

4. The biological information measurement instrument according to claim 2 or 3, wherein the light shielding film has a shape extending at least a predetermined distance or more from the openings to such an extent that light other than the light emitted from the light emitting unit is able to be shielded during the measurement instrument being attached to the human body.

5. The biological information measurement instrument according to any one of claims 2 to 4, wherein an impact absorbing material is provided on a back surface of the flexible substrate.

6. The biological information measurement instrument according to any one of claims 2 to 5, further comprising an output unit that outputs information on the light received by the light receiving unit.

7. The biological information measurement instrument according to any one of claims 1 to 6, wherein the recesses are formed by bending the flexible substrate.

# *FIG 1*

(a)

(b)

(c)

# FIG 2

# FIG 3

# FIG. 4

```
        ┌──────────────┐
        │    START     │
        └──────────────┘
               │
               ▼
                                          ⟋S401
┌─────────────────────────────────────────────┐
│       MAKE LIGHT EMITTING UNIT EMIT           │
└─────────────────────────────────────────────┘
               │
               ▼
                                          ⟋S402
┌─────────────────────────────────────────────┐
│         RECEIVE SENSING DATA FROM             │
│           LIGHT RECEIVING UNIT                │
└─────────────────────────────────────────────┘
               │
               ▼
                                          ⟋S403
┌─────────────────────────────────────────────┐
│   CALCULATE OXYGEN SATURATION AND             │
│   CONCENTRATION OF HEMOGLOBIN ON              │
│      THE BASIS OF SENSING DATA                │
└─────────────────────────────────────────────┘
               │
               ▼
                                          ⟋S404
┌─────────────────────────────────────────────┐
│        OUTPUT CALCULATED OXYGEN               │
│      SATURATION AND CONCENTRATION             │
└─────────────────────────────────────────────┘
               │
               ▼
        ┌──────────────┐
        │     END      │
        └──────────────┘
```

# FIG 5

(a)

(b)

(c)

# FIG. 6

# FIG 7

(a)

(b)

**INTERNATIONAL SEARCH REPORT**

International application No.

PCT/JP2016/080534

A. CLASSIFICATION OF SUBJECT MATTER
*A61B5/1455*(2006.01)i

According to International Patent Classification (IPC) or to both national classification and IPC

B. FIELDS SEARCHED

Minimum documentation searched (classification system followed by classification symbols)
A61B5/1455, A61B5/0245

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | US 2015/0126831 A1 (COVIDIEN LP), 07 May 2015 (07.05.2015), paragraphs [0037] to [0043]; fig. 3 to 4 (Family: none) | 1-7 |
| A | US 2013/0317330 A1 (COVIDIEN LP), 28 November 2013 (28.11.2013), paragraph [0031]; fig. 2 to 3 & US 2010/0249554 A1 & WO 2010/111127 A1 & EP 2410905 A1 & CA 2753017 A1 | 1-7 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
07 December 2016 (07.12.16)

Date of mailing of the international search report
20 December 2016 (20.12.16)

Name and mailing address of the ISA/
Japan Patent Office
3-4-3, Kasumigaseki, Chiyoda-ku,
Tokyo 100-8915, Japan

Authorized officer

Telephone No.

Form PCT/ISA/210 (second sheet) (January 2015)

## INTERNATIONAL SEARCH REPORT

International application No.

PCT/JP2016/080534

C (Continuation). DOCUMENTS CONSIDERED TO BE RELEVANT

| Category* | Citation of document, with indication, where appropriate, of the relevant passages | Relevant to claim No. |
|---|---|---|
| A | JP 2016-500290 A (CAS Medical Systems, Inc.), 12 January 2016 (12.01.2016), paragraphs [0013] to [0017], [0019] to [0027]; fig. 2 to 7 & US 2015/0308946 A1 paragraphs [0050] to [0054], [0056] to [0060]; fig. 2 to 7 & WO 2014/093342 A1 & EP 2928371 A1 | 1-7 |
| A | US 4865038 A (RICH David), 12 September 1989 (12.09.1989), column 2, line 48 to column 4, line 29; fig. 2 to 3 (Family: none) | 1-7 |
| A | JP 2011-519591 A (Nonin Medical, Inc.), 14 July 2011 (14.07.2011), paragraphs [0013] to [0014]; fig. 2 & US 2009/0259114 A1 paragraphs [0025] to [0026]; fig. 2 & WO 2009/128914 A1 & EP 2306892 A1 & CA 2724017 A1 | 1-7 |
| A | WO 2008/065699 A1 (Pioneer Corp.), 05 June 2008 (05.06.2008), paragraphs [0045] to [0047]; fig. 1 to 2 & US 2010/0056887 A1 paragraphs [0071] to [0073]; fig. 1 to 2 & EP 2087837 A1 | 1-7 |
| A | WO 2015/029965 A1 (Murata Mfg. Co., Ltd.), 05 March 2015 (05.03.2015), paragraphs [0024] to [0029]; fig. 1 & US 2016/0166162 A1 paragraphs [0035] to [0040]; fig. 1 | 1-7 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150126831 A1

- US 20130317330 A1